# EUROPEAN PATENT SPECIFICATION

(11) **EP 4 221 780 B1**
(45) Date of publication and mention of the grant of the patent: **28.08.2024**
(21) Application number: 21783254.2
(22) Date of filing: 27.09.2021
(51) Int. Cl.: A61M 5/14, A61M 5/142, A61M 5/145, A61M 5/168

(54) **MEDICAL INJECTION DEVICE WITH GAS EVACUATION**
MEDIZINISCHE INJEKTIONSVORRICHTUNG MIT GASEVAKUIERUNG
DISPOSITIF D'INJECTION MÉDICALE AVEC ÉVACUATION DU GAZ

(30) Priority: 30.09.2020 EP 20306133
(43) Date of publication of application: 09.08.2023
(73) Proprietor: Guerbet, 93420 Villepinte (FR)
(72) Inventor: ALLARD, Ludovic, 69390 MILLERY (FR); CACLIN, Jérôme, 69360 SAINT SYMPHORIEN D'OZON (FR)
(74) Representative: Regimbeau
(86) International application number: PCT/EP2021/076464
(87) International publication number: WO 2022/069394

(56) References cited:
- US-A1- 2005 215 850
- US-A1- 2006 249 541
- US-A1- 2012 209 111

## Description

### TECHNICAL FIELD

The present invention generally relates to the injection of medical fluids and, more particularly, to an injection device for injecting into a medical tubing a medical liquid from a medical liquid container.

### BACKGROUND OF THE INVENTION

Injection devices are commonly used for injecting into a medical tubing a medical liquid from a medical liquid container. For example, the injection of medical liquid such as iodinated contrast agent is required in 70% of CT scan diagnosis cases. This injection, in about 70% of cases, is performed using an automated contrast agent injector. Injection tubing is required to connect the automated injector to the patient.

Patent application US20120209111 discloses a bladder syringe for a fluid delivery system which includes a cylindrical body, a cap-bladder assembly, a plunger element disposed in the cylindrical body, and a mounting assembly to secure the cap-bladder assembly to the cylindrical body. The cylindrical body has a distal end and a proximal end and defines a throughbore. The cap-bladder assembly is adapted for connection to the distal end of the cylindrical body, and includes a cap body and a bladder. The cap body defines an interior cavity and a distal discharge conduit and is adapted to engage the distal end of the cylindrical body. A disc-shaped bladder is disposed within the interior cavity and typically includes a central membrane portion. The plunger element is disposed in the throughbore of the cylindrical body and is vented to enable evacuation of the space between the plunger element and the cap-bladder assembly in the cylindrical body.

Patent application US20060249541 discloses a fluid dispensing device includes a bottle for receiving fluid, a discharge tube, and a pressurizing device coupled between the bottle and the discharge tube, for pressurizing the fluid and for forcing the fluid to flow through the discharge tube without gravity. The pressurizing device includes a container coupled between the bottle and the discharge tube, a piston slidably received in the container, and a moving device for moving the piston in a reciprocating action within the container. For example, a motor is coupled to the piston with a crank, to move the piston in the reciprocating action within the container.

Patent application US20050215850 discloses A syringe pump including a syringe including a plunger that slides in a body which has a discharge port, a driving mechanism coupled to the syringe, including a cylinder in which a piston mounted on a shaft slides, and a biasing device operative to apply an urging force on the piston to drive the piston distally in the cylinder, and a safety catch that initially prevents the biasing device from moving the piston, the safety catch being removable to permit the biasing device to move the piston.

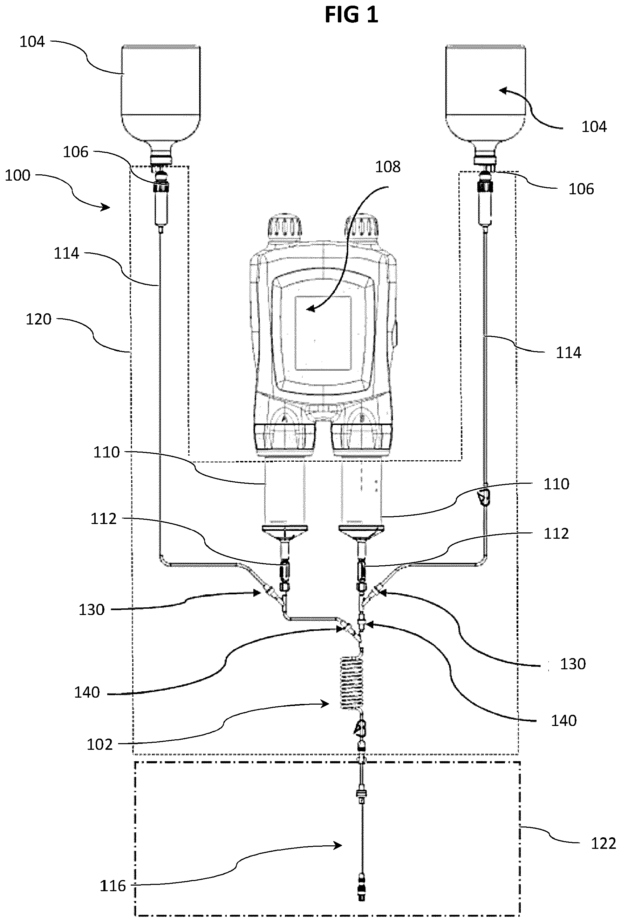

**Figure 1** shows an example of an injection system 100 for injecting a medical liquid from a medical liquid container 104 into a common line 102. Those skilled in the art will appreciate the medical liquid container 104 can be any type of container specifically adapted for containing medical liquids, for example, such as, but not limited to: vials, bottles, plastic containers, and any type of container manufactured to contain a medical liquid. The injection system comprises a first connector 106 configured to be connected to a medical liquid container 104, an injector 108 to which is attached an injection device 110 with a medical tubing interface 112, a medical liquid supply line 114 configured to connect the first connector 106 to the medical tubing interface 112 for supplying the medical liquid to the injection device 110, and a common line 102 configured to be connected to the medical tubing interface 112 and to a patient line 116 for injecting the medical liquid into the patient line 116. More precisely, a first tubing valve 130 connects the liquid supply line 114 to common line 102 and allows only passage to the common line 102 under a vacuum pressure. The common line 102 comprises a second tubing valve 140 downstream of the first tubing valve 130 and only authorizes passage towards the patient line 102. The injection device 110 usually comprises a body defining an inner space, and a piston driven by a plunger rod and configured for travelling within the inner space to pump fluid into the inner space or push the fluid out of the inner space.

In the depicted example, two different types of medical liquids are to be injected into the patient, and consequently, the injection system is configured to be connected to two medical liquid containers 104, with two different first connectors 106 and two different medical liquid supply lines 114. However, the injection system may be configured to inject only one medical liquid. For simplicity's sake, the following description will be made with reference to a configuration where only one medical liquid is to be injected, since bi-injection merely involves replicating the described features. Here "bi-injection" is understood to mean two-injections.

For economic and ecological reasons (less use of plastic), the multi-patient practice is steadily gaining market share. In the so-called multi-patient practice, the tubing for the injection system is provided with two very distinct parts: the day set 120 and the patient set 122. The patient set 122 is changed for each patient. The patient set 122 is typically used to limit risks of cross contamination between consecutive patients and thus protects the day set. Once installed and primed the day set 120 stays connected on the power injector for several patient cases, as long as the same medical fluid is to be injected. When the medical fluid to be injected needs to be changed, then the day set 120 is changed. This day set 120 comprises the medical liquid supply line 114 which is connected to the medical liquid container 104 and to the common line 102. The patient set 122 comprises the patient line 116 which is supplied in medical liquid by the common line 102 and is connected to a catheter or a needle for injecting the medical liquid into the patient.

When the injection system 100 is set up for injecting medical liquid into a patient, it is important to ensure that no gas is present in the tubing before injection. Injecting a gas such as air into a blood vessel of a patient may result in a gas embolism, i.e. a blood vessel blockage caused by one or more bubbles of air or other gas in the circulatory system. When the day set 120 or the patient set 116 is put in place, the tubes are filled with air. It is therefore necessary to evacuate the gas present in the tubes before injection. Due to the length of the tubes, a large quantity of gas is to be evacuated from the injection system 100.

For purging the injection system 100 from all the gas present before the injection, the injection device 110 draws or pulls medical liquid from the medical liquid container 104, thereby filling the medical liquid supply line 114. The injection device 110 is now filled with a mixture of medical liquid and gas. The injection device 110 is then positioned with the medical tubing interface 112 upwards so that the gas is gathered at said medical tubing interface 112. It should be noted that the filling of the injection device 110 causes a turbulent flow of medical liquid, generating micro-bubbles in the medical liquid. Due to the high viscosity of the medical liquid (especially for contrast agent), the micro-bubbles may need several minutes for reaching the medical tubing interface 112. It is therefore customary to wait at least 2 or 3 minutes with the medical tubing interface 112 upwards. Then, by actuating the piston, the gas is evacuated from the injection device 110 through the still upwards medical tubing interface 112, into the common line 102. Medical liquid is then injected into the common line 102 for pushing the gas outside the common line 102, thereby purging the injection system 100.

During injection, it may happen that gas is present in the injection device 110. For example, vaporization of the medical liquid may create gas. Also, some bubbles generated during the initial filling of the injection system 100 may be blocked in the tubing or against the walls of the injection device 110 and may not be evacuated during the initial purge. As a result, during injection, the injection device 110 is positioned with the medical tubing interface 112 downwards, so that any gas present in the injection device 100 will be trapped in the injection device 100 away from the medical tubing interface 112, and will not be injected into the common line 102.

This approach suffers from several drawbacks. Firstly, the injection device 110 must be moved between two opposite positions: with the medical tubing interface 112 upwards or downwards. This requires that the injector 108 is able to rotate. Secondly, this purge takes a significant time, and the injection system 100 must be monitored by an operator during the purge. The operator must also assess the quality of the purge and whether the purge is completed or not. As with every human interaction, reliance on an operator may lead to errors. Thirdly, the gas is pushed along the common line 102 by medical liquid which also exits the injection system. This approach thereby involves wasting medical liquid, and requires collecting the wasted medical liquid at the output of the injection system 100, with possible handling error.

Also, gas still present in the injection device 110 after the purge may alter the operating of the injection device, even if the gas is trapped in the injection device 110. Dosage of the medical liquid is usually controlled through the course of the piston of the injection device 110. Gas is compressible, and the volume variation of medical fluid within the injection device will therefore be inaccurate. Also, the volume of gas trapped in the injection device 110 must be small, otherwise it risks being injected into the common line 102.

Accordingly, there is a need for an injection system able to evacuate the gas without requiring any operator's involvement, rapidly and each time gas is present in the injection device.

### SUMMARY OF THE INVENTION

It is proposed an injection device for injecting into a medical tubing a medical liquid from a medical liquid container, said injection device comprising:
- a body defining an inner space extending in a longitudinal direction between an upper end of the body and a lower end of the body, the lower end of the body comprising a medical tubing interface through which the medical liquid can penetrate the inner space from the medical liquid container and can exit said inner space to be injected into the medical tubing,
- a piston arranged within the inner space and configured for travelling within the inner space along the longitudinal direction, said piston delimiting an upper space and a lower space of the inner space, said lower space configured to receive the medical liquid,
- an evacuation path, said evacuation path traversing the piston in the longitudinal direction from the lower space to the upper space of the inner space,
- a selector arranged within the evacuation path between a lower portion of the evacuation path and an intermediate portion of the evacuation path, said lower portion of the evacuation path being connected to the lower space, wherein the selector is configured for selectively allowing the gas to go through said selector and to travel along the evacuation path from the lower portion to the intermediate portion of the evacuation path, said selector configured for selectively preventing the medical liquid from going through said selector and from travelling along the evacuation path from the lower space to the upper space of the inner space,
- a purge valve arranged in the evacuation path between the intermediate portion of the evacuation path and an upper portion of the evacuation path and configured for moving between a blocking configuration in which the purge valve closes the evacuation path and a passing configuration in which the purge valve keeps open said evacuation path, wherein the passing configuration of the purge valve requires an overpressure in the intermediate portion of the evacuation path caused by the piston travelling towards the lower end of the body.

The invention allows evacuating the gas from the injection device each time the piston travels for pushing medical fluid into the medical tubing. The purge is therefore performed automatically, without requiring any operator's intervention, and ensures that no gas is injected into the medical tubing.

Other preferred, although non-limitative, aspects of the invention are as follows, isolated or in a technically feasible combination:
- the purge valve is configured for being in the blocking configuration when the piston travels towards the upper end of the body;
- the lower portion of the evacuation path is configured for receiving both medical liquid and a gas, and the intermediate portion and the upper portion of the evacuation path are configured for receiving only gas;
- the upper space is configured to be kept at a constant reference pressure and the overpressure in the intermediate portion of the evacuation path corresponds to a gas pressure above the reference pressure;
- the piston comprises a lower interface delimiting the lower space of the inner space, said lower interface comprising an entrance of the evacuation path which opens in a highest portion of said lower interface;
- the lower interface has a convex surface seen from the lower space of the inner space and the entrance of the evacuation path opens in a middle of said lower interface or the lower interface has a concave surface seen from the lower space of the inner space and the entrance of the evacuation path opens in a periphery of said lower interface;
- the selector is a float configured for floating on the medical liquid and arranged in a cavity, said cavity comprising at least a passage which belongs to the evacuation path, wherein said float is configured for travelling within said cavity along the longitudinal direction between a blocking configuration in which the float obturates the passage, thereby closing the evacuation path, and an open configuration in which the float is spaced apart from the passage, thereby letting open the evacuation path;
- the float has at least one obturating part and a floating part, said obturating part configured to obturate the passage, wherein the floating part has an enlarged section with respect to a widest section of the obturating part;
- the float is configured to obturate the passage with two distinct zones contacting a seat defining the passage;
- the selector is a membrane extending across the evacuation path, said membrane being hydrophobic and configured for letting the gas passes through, and for preventing the medical liquid from passing through;
- the membrane is arranged in a cavity, and a support organ is arranged in said cavity against an upper side of the membrane to mechanically support the membrane, said support organ allowing the gas passes through the membrane and through said support organ.

The invention also relates to an injection system comprising:
- an injection device as described in any embodiment,
- a first connector configured to be connected to a medical liquid container,
- a medical liquid supply line configured to connect the first connector to the medical tubing interface for supplying the medical liquid to the injection system,
- a common line configured to be connected to the medical tubing interface and to a patient line for injecting the medical liquid into the patient line.

Preferably, the overpressure corresponds to a pressure in the intermediate portion of the evacuation path exceeding a reference pressure in the upper space by at least a first pressure threshold, and
wherein the common line comprises a threshold valve with a second pressure threshold,
wherein the first pressure threshold is inferior to the second pressure threshold.

The invention also relates to a process for operating an injection device as described in any embodiment, wherein the injection device is kept with the upper end of the body upwards, and the lower end of the body downwards, the process comprising:
- a filling step, wherein the piston travels within the inner space along the longitudinal direction towards the upper end of the body, causing the medical liquid from the medical liquid container and gas to penetrate the inner space through the medical tubing interface, wherein the purge valve is in the blocking configuration,
- a purge step, wherein the piston travels within the inner space along the longitudinal direction towards the lower end of the body, and wherein the gas is evacuated from the lower space to the upper space of the inner space through the evacuation path traversing the piston, while the selector keeps the medical liquid in the lower space, wherein the purge valve is in the passing configuration.

### BRIEF DESCRIPTION OF THE DRAWINGS

Other aspects, objects and advantages of the present invention will become better apparent upon reading the following detailed description of preferred embodiments thereof, given as non-limiting examples, and made with reference to the appended drawings wherein:
- Figure 1 is a general view of an injection system with two medical liquid containers connected to it;
- Figure 2 is a cross-sectional view of a piston of an injection device according to a non-limiting possible embodiment wherein the selector is a float, during a filling step before the purge;
- Figure 3 is a cross-sectional view of a piston of an injection device according to a non-limiting possible embodiment wherein the selector is a float, during the beginning of the purge;
- Figure 4 is a cross-sectional view of a piston of an injection device according to a non-limiting possible embodiment wherein the selector is a float, at the end of the purge;
- Figure 5 is a cross-sectional view of a piston of an injection device according to a non-limiting possible embodiment wherein the selector is a float, during the injection step after the purge;
- Figure 6 is a cross-sectional view of a float acting as a selector, according to non-limiting possible embodiments;
- Figures 7a, 7b, 7c are cross-sectional views of a float of an injection device according to a non-limiting possible embodiment, during a two-stage obturation of the evacuation path;
- Figure 8 is a cross-sectional view of a piston of an injection device according to a non-limiting possible embodiment wherein the selector is a membrane, during a filling step before the purge;
- Figure 9 is a cross-sectional view of a piston of an injection device according to a non-limiting possible embodiment wherein the selector is a membrane, during the beginning of the purge;
- Figure 10 is a cross-sectional view of a piston of an injection device according to a non-limiting possible embodiment wherein the selector is a membrane, at the end of the purge;
- Figure 11 is a cross-sectional view of a piston of an injection device according to a non-limiting possible embodiment wherein the selector is a membrane, during the injection step after the purge.

### DETAILED DESCRIPTION OF THE INVENTION

The injection device of the invention can be used in an injection system 100 as previously described in relation with **Figure 1****.** The injection system 100 will not be described any further, except for the injection device 1.

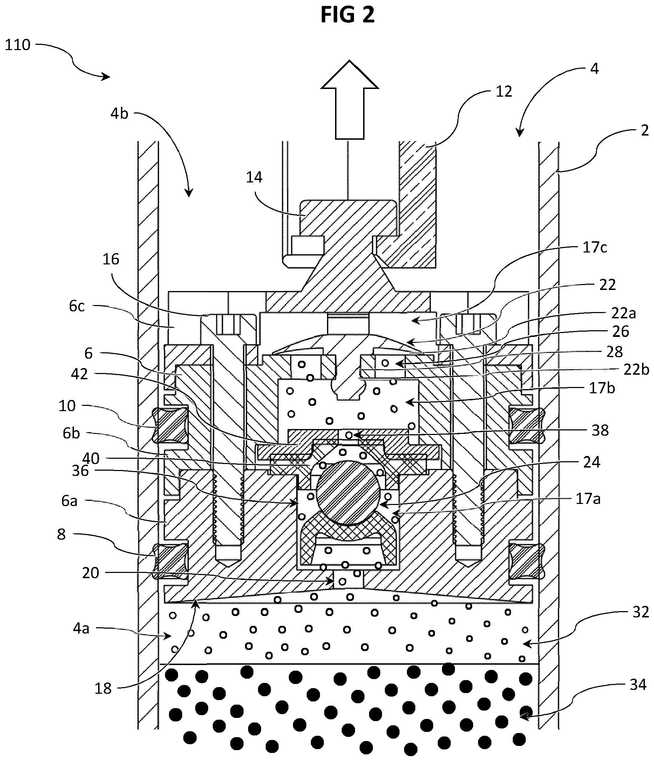

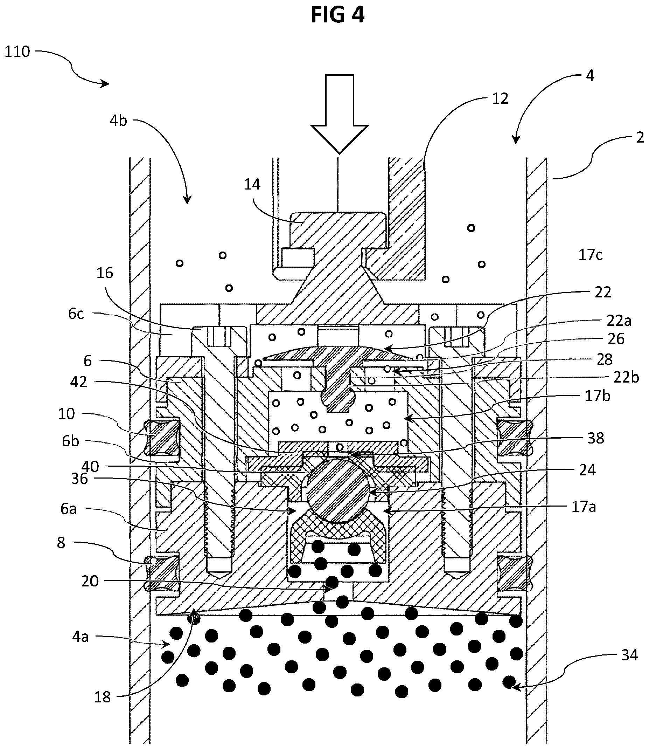

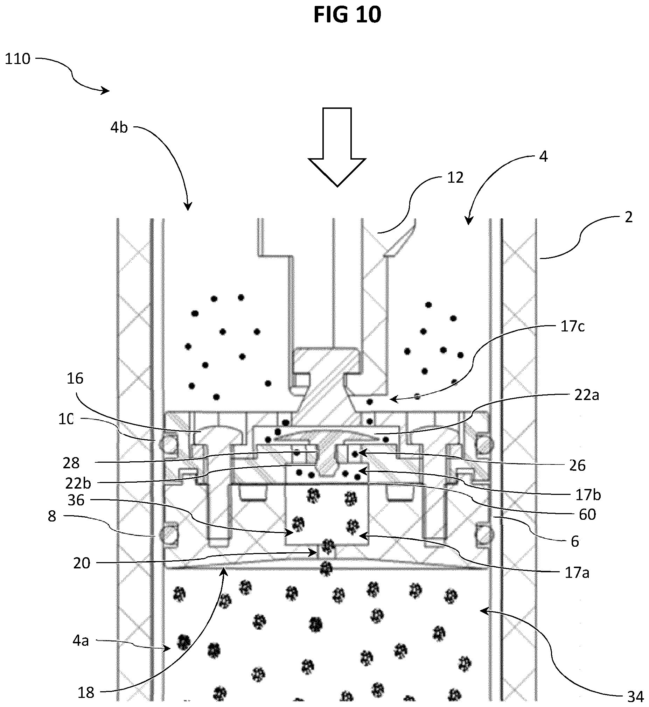

With reference to **Figures 2-5****,** and **8-11,** the injection device 110 includes a body 2 defining an inner space 4 extending in a longitudinal direction between an upper end of the body 2 and a lower end of the body 2, the lower end of the body having a medical tubing interface 112 through which the medical liquid can penetrate the inner space 4 from the medical liquid container 104 and can exit said inner space 4 to be injected into the common line 102. Contrary to previous injection devices, the injection device 110 according to the invention is configured for staying with the medical tubing interface 112 downwards. Spatial terms such as "downwards", "upwards", "lower", "upper", "higher", "highest" must be understood as defining the commonly accepted relative position with respect to the vertical, i.e. the local Earth gravity direction. This is because gas and medical liquid are subjected to the gravity, and the present invention makes use of the gravity for a proper gas evacuation. As a result, in use, the lower end of the body 2 is located below the upper end of the body 2. The body 2 is typically a hollow cylindric body, for example made of glass or plastic material, also called a barrel.

The injection device 110 also includes a piston 6 arranged within the inner space 4 and configured for travelling within the inner space 4 along the longitudinal direction, i.e. between the upper end of the body 2 and the lower end of the body 2. The piston 6 delimits an upper space 4b and a lower space 4a of the inner space. The lower space 4a is configured to receive the medical liquid, whereas the upper space 4b is not intended to receive any liquid. The piston 6 provides hermetic sealing between the upper space 4b and a lower space 4a. To this end, the piston 6 is provided with at least one peripheral seal 8, 10, for example made of rubber, and preferably two peripheral seals 8, 10 at different height along the longitudinal direction. Each peripheral seal 8, 10 is pressed against the wall of the body 2, ensuring tight sealing. As in the depicted example, a peripheral seal 8, 10 can be a quad-ring, but can also be for example O-ring.

Due to the tight sealing provided by the piston 6, pressure can significantly differ between upper space 4b and a lower space 4a of the inner space 4. The pressure within the upper space 4b is kept at a reference pressure which is roughly constant and substantially independent from the course of the piston 6. This reference pressure is typically the atmospheric pressure, e.g. the pressure of the system's environment. Preferably, the upper end of the body 2 is at least partially open so that the pressure inside the upper space 4b corresponds to the atmospheric pressure, regardless of the course of the piston 6. On the opposite, the pressure inside the lower space 4a of the inner space 4 depends on the content of said lower space 4a and on the course of the piston 6. In the description below, an overpressure is a pressure above the reference pressure, and a vacuum pressure is a pressure below the reference pressure.

The piston 6 is attached to a piston rod 12, for example by a protrusion 14 on top of the piston 6 which is engaged into said piston rod 12. The piston rod 14 is driven by the injector 108 and causes the piston 6 to travel within the inner space 4 along the longitudinal direction. The piston 6 can be formed by several parts assembled together. In the depicted example, the piston 6 has a lower part 6a, an intermediate part 6b and an upper part 6c. Connectors such as screws 16 may be used for assembling the parts of the piston.

The piston 6 has an evacuation path arranged within the piston 6. The path traverses the piston 6 in the longitudinal direction from the lower space 4a to the upper space 4b of the inner space 4. The evacuation path is meant to evacuate into the upper space 4b the gas present in the lower space 4a. Typically, the evacuation path is not straight and can be closed or open at different points by components of the piston 6, as described below. More specifically, the evacuation path includes a lower portion 17a, an intermediate portion 17b, and an upper portion 17c. The lower portion 17a of the evacuation path is connected to the lower space 4a, whereas the upper portion 17c of the evacuation path is connected to the upper space 4c. The intermediate portion 17b is between the lower portion 17a and the upper portion.

The piston 6 includes a lower interface 18 delimiting the lower space 4a of the inner space 4, said lower interface 18 having an entrance 20 of the evacuation path. Preferably, the entrance 20 opens in a highest portion of the lower interface 18 in order to properly evacuate all the gas present in the lower space, without any gas being trapped in the lower space 4a of the inner space, against the lower interface 18. Preferably, the lower interface 18 has a surface with an apex pointing towards the upper end of the body, and the entrance 20 of the evacuation path opens at said apex. For example, the lower interface 18 has a convex surface seen from the lower space 4a of the inner space, and the entrance 20 of the evacuation path opens in a middle of said lower interface 18, as in the depicted example. For instance, the surface of the lower interface 18 may correspond to a surface of a cone, a truncated cone, or a pyramid pointing upwards. Alternatively, the lower interface 18 may have a concave surface seen from the lower space 4a of the inner space and the entrance 20 of the evacuation path opens in a periphery of said lower interface 18. For example, the lower interface 18 may have a groove arranged in a periphery of the lower interface 18, and the entrance 20 of the evacuation path may open in said groove.

The piston 6 includes a purge valve 22 arranged in the evacuation path between the intermediate portion 17b of the evacuation path and the upper portion 17c of the evacuation path. The purge valve 22 is configured for moving between a blocking configuration in which the purge valve 22 closes the evacuation path, and a passing configuration in which the purge valve 22 keeps open said evacuation path. The passing configuration of the purge valve 22 requires an overpressure in the intermediate portion 17b of the evacuation path with respect to the reference pressure in the upper space 4b, caused by the piston 6 travelling towards the lower end of the body 2. Since the upper space 4b is at the reference pressure (e.g. the atmospheric pressure), the overpressure means a pressure above the reference pressure. More precisely, the overpressure required for the passing configuration of the purge valve 22 corresponds to a pressure in the intermediate portion 17b exceeding the reference pressure in the upper space 4b and the upper portion 17c of the evacuation path by at least a first pressure threshold. The purge valve 22 is configured for being in the blocking configuration when the piston 6 travels towards the upper end of the body 2 since there is no overpressure in the intermediate portion 17a with respect to the reference pressure in the upper space 4b, but instead a vacuum pressure, i.e. a pressure below the reference pressure.

The piston 6 also includes a selector 24, 60 arranged within the evacuation path between the lower portion 17a of the evacuation path and the intermediate portion 17b of the evacuation path. The selector 24, 60 is configured for selectively allowing the gas to go through said selector 24, 60 and to travel along the evacuation path from the lower portion 17a to the intermediate portion 17b of the evacuation path. The selector 24, 60 is also configured for selectively preventing the medical liquid from going through said selector 24, 60 and from travelling along the evacuation path from the lower portion 17a to the intermediate portion 17b of the evacuation path, and consequently from travelling along the evacuation path from the lower space 4a to the upper space 4b of the inner space. As a result, the lower portion 17a of the evacuation path is a mixed portion configured for receiving both medical liquid and gas, and the intermediate portion 17b and upper portion 17c are gaseous portions configured for receiving only gas.

The purge valve 22 is arranged upper than the selector 24, 60 in the longitudinal direction from the lower space 4a to the upper space 4b of the inner space. The purge valve 22 is thus arranged in the gaseous portion of the evacuation path and is not in contact with any liquid.

In the blocking configuration, the purge valve 22 seals a vent 26 between the intermediate portion 17b and the upper portion 17c of the evacuation path. In the passing configuration, the purge valve 22 lets said vent 26 open. In the depicted example, two vents 26 appear between the intermediate portion 17b and the upper portion 17c of the evacuation path. More or fewer vents 26 can be provided, as long as they are sealable by the purge valve 22.

Preferably, as depicted in the illustrated embodiment, the purge valve 22 is an umbrella valve, which has a diaphragm shaped sealing disk 22a and a stem 22b. The stem 22b is engaged into a hole 28 arranged in a fixed part of the piston 6 and presents an enlarged lower portion with a section superior than the section of the hole 28, thereby affixing the purge valve 22. The diaphragm shaped sealing disk 22a is disposed above at least one vent 26 which is part of the evacuation path and delimits the intermediate portion 17c from the upper portion 17c of the evacuation path. The umbrella valve can be deformable and/or can slidably move along the hole 28 arranged in the fixed part of the piston 6 to change configuration (for example due to the deformation of the stem). In the passing configuration, the sealing disk 22a is away from the vent 26 because of a higher gas pressure within the intermediate portion 17b, thereby allowing gas passing through said vent 26. In the blocking configuration, the sealing disk 22a is pressed against the vent 26 because of a higher gas pressure within the upper portion 17c of the evacuation path, thereby sealing said vent 26, and closing the evacuation path. For example, the umbrella valve can be made of elastomer of rubber type, or in silicon.

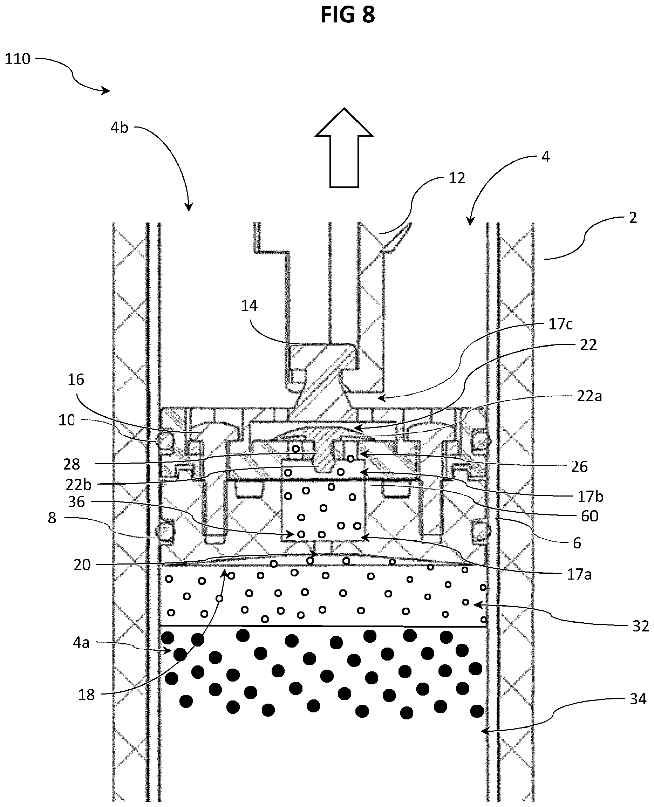

The selector 24, 60 can be for example a float 24 configured for floating on the medical liquid or a hydrophobic membrane 60. A process for operating an injection device with a float 24 as a selector will now be described with reference to **Figures 2-7****,** and later a process for operating an injection device with a membrane 60 as a selector will be described later with reference to **Figures 8-11****.** Except for the selector 24, 60, the features of the injection devices 110 and of the processes described below can apply to any embodiment.

### The selector is a float

In **Figure 2****,** the injection device 110 is shown before the purge, for instance during the filling step wherein the injection device 110 is filled with medical liquid and unwanted gas. During this filling step, the piston rod 12 is driven upwards, for example by the injector 108 acting on said piston rod 12, thereby causing the piston 6 to travel within the inner space 4 along the longitudinal direction towards the upper end of the body 2, i.e. upwards. The lower space 4a expands, resulting in a gas pressure within said lower space 4a to drop below the reference gas pressure within the upper space 4b, e.g. the atmospheric pressure.

Since the selector 24 allows gas to travel from the lower portion 17a to the intermediate portion 17b of the evacuation path, the decreased pressure in the lower space 4a is also found in the lower portion 17a and the intermediate portion 17b of the evacuation path. However, as the purge valve 22 requires an overpressure in the intermediate portion 17b of the evacuation path to be in the passing configuration, the purge valve 22 is kept in the blocking configuration. More precisely, the purge valve 22 is pressed downwards, sealing the vent 26 and thereby closing the evacuation path in the blocking configuration.

The expansion of the lower space 4a combined with the closing of the evacuation path by the purge valve 22 effectively creates a vacuum pressure in the lower space 4a, i.e. a pressure below the reference pressure. The pressure in the lower space 4a decreases until reaching the opening pressure of the first tubing valve 130, which is, for example, comprised between 0.2 and 0.5 bars below the reference pressure. The opening of the first tubing valve 130 creates an aspiration of the medical liquid to compensate this vacuum pressure in the lower space 4a. The medical liquid travels through the filling line 114 connected to the medical liquid container 104 to fill the lower space 4a. Gas present in the tubing is also aspirated into the lower space 4a.

Progressively, as the lower space 4a becomes filled with medical liquid and gas, the pressure within the lower space 4a increases and becomes closer to the atmospheric pressure. When the pressure within the lower space 4a reaches the closing pressure of the first tubing valve 130 (substantially similar to the opening pressure), the first tubing valve 130 closes and the filling is stopped. At the end of the filling step, the lower space 4a is filled with a volume of gas 32 above a volume of medical liquid 34. The gas pressure within the lower space 4a is still below the gas pressure within the upper space 4b because the pressure increase was stopped by the closing of the first tubing valve 130 before the vacuum pressure had been completely compensated. Consequently, the purge valve 22 stays in the blocking configuration.

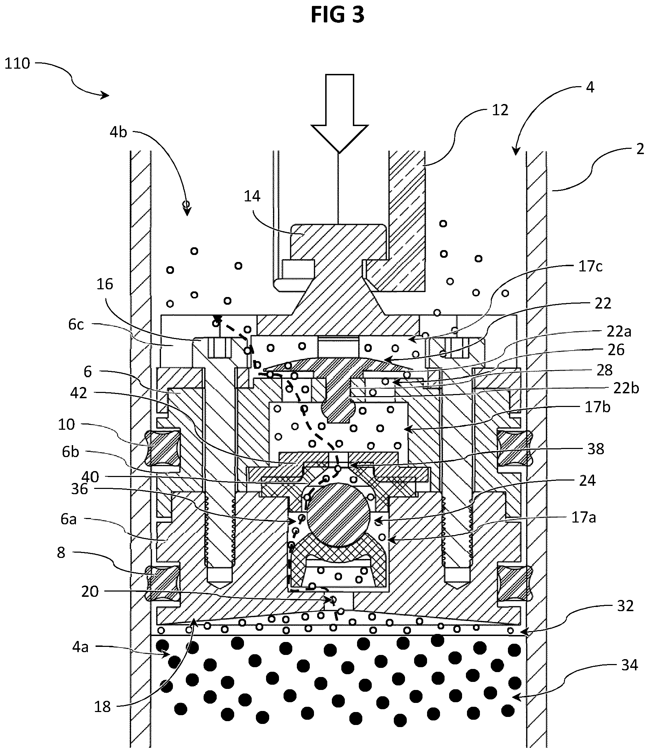

As explained above, after the filling of the injection device 110, the gas in the lower space 4a must be evacuated during a purge. This purge is performed by driving the piston 6 downwards, as shown on **Figure 3****.** The piston 6 travels within the inner space 4 along the longitudinal direction towards the lower end of the body 2. The lower space 4a shrinks, and gas pressure increases in the lower space 4a until the pressure difference between the gas pressure within the intermediate portion 17b, still communicating with the lower space 4a, and the reference gas pressure within the upper space 4b becomes higher than the opening pressure threshold of the purge valve 22, i.e. until the pressure difference reaches the first pressure threshold.

As indicated above, the purge valve 22 is configured to move to the passing configuration in response to an overpressure in the lower space 4a exceeding the reference pressure in the upper space 4b by at least the opening pressure threshold of the purge valve 22 (for example between 20 and 100 mbars of pressure difference between the overpressure and the reference pressure). The purge valve 22 therefore moves to the passing configuration, thereby opening the evacuation path. In the depicted example, the sealing disk 22a moves away from the vents 26, thereby unsealing said vents 26.

The gas is evacuated from the lower space 4a to the upper space 4b of the inner space 4 through the evacuation path traversing the piston 6. More specifically, gas enters the evacuation path through the entrance 20, then travels along the lower portion 17a of the evacuation path, then along the intermediate portion 17b of the evacuation path, then through the vents 26 and finally along the upper portion 17c to reach the upper space. This is shown by the dotted arrow in **Figure 3****.**

As the piston 6 travels downwards while the gas is evacuated through the evacuation path, the piston reaches the volume of medical liquid in the lower space 4a. More specifically, the lower interface 18 contacts the medical liquid, and the gas is pushed back towards the entrance 20 of the evacuation path since the entrance 20 opens in a highest portion of said lower interface 18. The gas is therefore evacuated from the lower space 4a before the medical liquid reaches the entrance 20 of the evacuation path. When all the gas has been evacuated, the medical liquid penetrates through the entrance 20 in the lower interface 18 of the piston 6 and fills the lower portion 17a of the evacuation path.

As illustrated, the lower portion 17a of the evacuation path can include a cavity 36 where the selector 24 is arranged and the medical liquid begins filling the cavity 36. The selector is a float 24 configured for floating on the medical liquid and the cavity 36 is configured to allow the float 24 to travel up and down the cavity 36, along the longitudinal direction. The cavity 36 includes at least a passage 38 forming the boundary between the lower portion 17a and the intermediate portion 17b of the evacuation path. The passage 38 is arranged at the top of the cavity 36. The float 24 is configured for travelling within the cavity 36 along the longitudinal direction between a blocking configuration in which the float 24 obturates the passage 38, thereby closing the evacuation path, and an open configuration in which the float 24 is spaced apart from the passage 36, thereby keeping open and unblocked the evacuation path.

More precisely, when the cavity 36 is filled with gas 32, the float 24 stays at the bottom of the cavity 36, keeping open the passage 38 and thereby keeping the evacuation path unblocked. When the medical liquid reaches the cavity 36, the float 24 begins floating on the medical liquid, and therefore moves up, carried by the medical liquid 34 in accordance with the upward buoyant force that is exerted on the float by the medical liquid (Archimedes' principle). Under this force, the float 24 travels upward until reaching the top of the cavity 36 and blocks the passage 38.

The passage 38 is defined by a periphery forming a seat 40 for the float 24, facing said float 24. The float obturates the passage 38 by pressing against the seat. The seat 40 is for example made of metal or plastic such as thermoplastic polyurethane, polyoxymethylene, polycarbonate, polyvinyl chloride, etc.. Advantageously, the seat 40 is made from a material with an elastic modulus higher than 2500 Mpa (megapascals). Preferably, the seat 40 has a decreasing section in the direction of the intermediate portion 17b of the evacuation path and, for example, the shape of the seat 40 is, at least partially, a hollow truncated cone. A reinforcing element 42, such as a washer, can be provided above the seat 40 to strengthen the seat, especially when said seat is made in a highly deformable material.

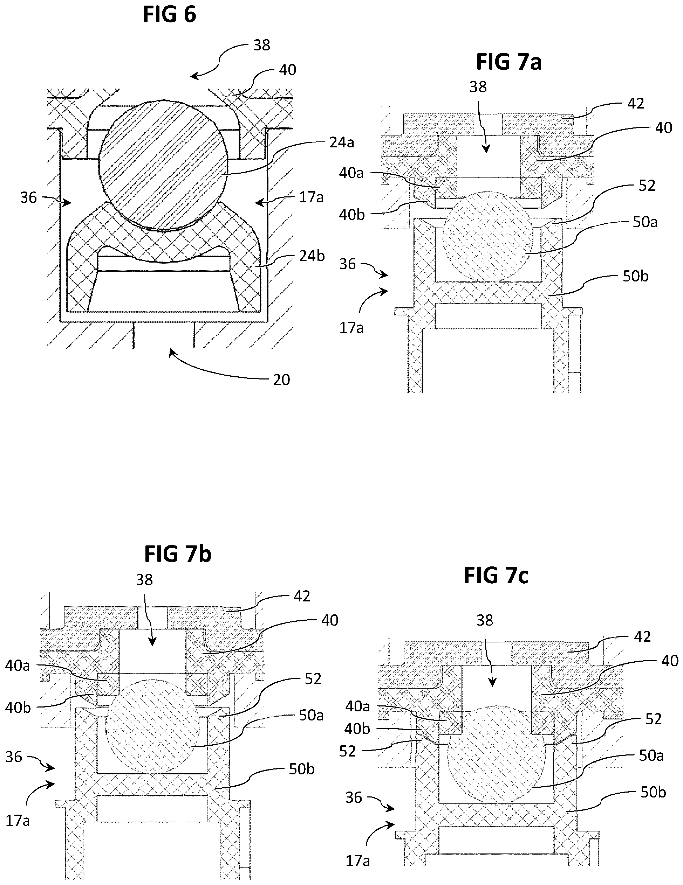

As in the depicted example of **Figure 6****,** the float 24 has at least an obturating part 24a configured to obturate the passage 38. Typically, the passage 38 has a round section and the obturating part 24a of the float 24 has a round section too. For example, the obturating part 24a of the float 10 may have at least partially an ellipsoid shape, or a spherical shape or a conical shape like a pine. For example, the float 24 can be a mere ball. Preferably, the obturating part 24 of the float is coated with a deformable material for a better sealing of the passage 38 when the float 24 is pressed against the seat 40.

Preferably, the float 24a has a floating part 24b with an enlarged section with respect to a widest section of the obturating part 24a, said floating part 24b supporting the obturating part 24a. The float can be in two-parts as in **Figure 6** or can be in a single piece including the obturating part 24a and the floating part 24b. The obturating part 24a of the float 24 is for example a ball. The floating part 24b of the float 24 has a diameter superior to the widest diameter of the obturating part 24a of the float, i.e. the ball portion. The higher diameter of the floating part 24b of the float 24 (perpendicularly to the longitudinal direction) makes the float 24 more responsive to a low force exerted by the medical liquid 34 on the float 24. This enhanced response allows pushing the float 24 upwards as soon as medical liquid reaches the float 24 and therefore ensures that no medical liquid can reach the passage 38 before said passage is obturated by the float 24.

When the medical liquid 34 contacts the float 24, e.g. the floating part 24b of the float 24, the float 24 begins floating and therefore is moved upwards until it reaches the seat 40. The shape of the float 24 and the shape of the seat 40 are adapted so that a continuous sealing is created when the float 24 presses the seat 40. The passage 38 is now obturated, and neither gas or medical liquid can pass through the obturated passage 38. A small volume of gas may be trapped within the cavity 36, i.e. in the lower portion of the evacuation path, between the obturated passage 38 and the surface of the medical liquid 34. This small volume enables the seat to be kept dry and to avoid any material deposition on the seat 40. The small volume, for example, may be a volume less than 10 ml at atmospheric pressure. This small volume of air may be a sufficient quantity to dry the float 24. The density of the float 24 and the complementary shapes of the obturating part 24a and of the seat 40 are chosen to minimize the volume of trapped air, while keeping the surface of medical liquid away from the seat 40. For example, the seat's section may decrease in the direction of the intermediate portion 17b of the evacuation path.

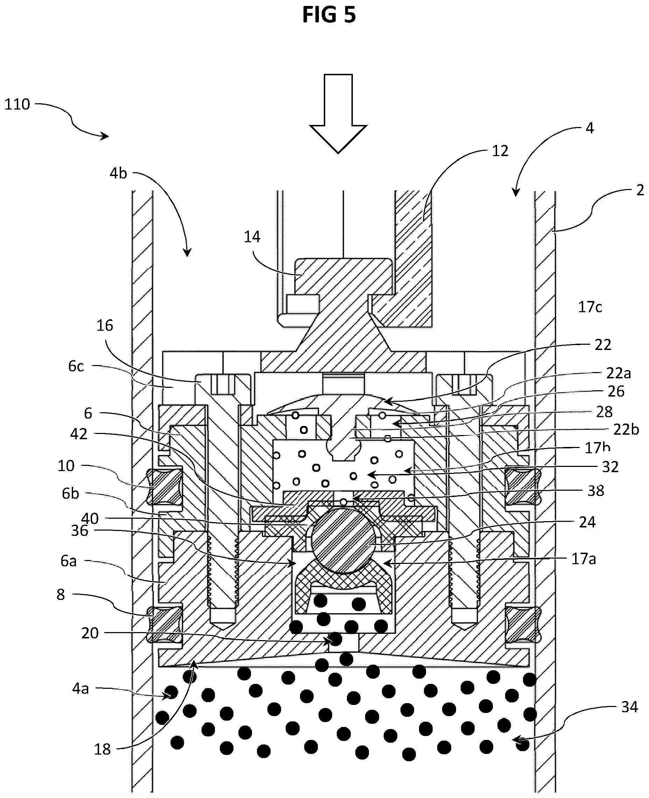

Once the passage 38 is obturated, no gas escapes the overpressure in the lower portion 17a of the evacuation path to reach the intermediate portion 17b. As a result, the pressure within the intermediate portion 17b above the obturated passage 38 drops until the difference between the pressure in the intermediate portion 17b and the reference pressure reaches the closing pressure threshold of the purge valve 22, which is slightly above the reference pressure since the upper space 4b is at said reference pressure. For example, the closing pressure threshold of the purge valve 22 can correspond to a positive pressure difference of 20 to 100 mbars between the pressure within the intermediate portion 17b and the reference pressure. Preferably, the closing pressure threshold and the opening closing threshold are substantially the same, but they can also differ. As a result, the purge valve 22 is now closed **(****Figure 5****)** in the blocking configuration. In this example, the sealing disk 22a coves the vent 26. The purge step is over. A small volume of gas 32 at a residual overpressure (i.e. slightly above the reference pressure) is kept in the intermediate portion 17b. The residual overpressure is not high enough to cause the purge valve 22 to open the vents 26. This residual overpressure ensures that air at the atmospheric pressure coming from the upper space 4b cannot penetrate the intermediate portion 17b of the evacuation path, thus avoiding non-sterile air from entering the sterile area where the medical liquid circulates.

The process may include a complementary filling step which occurs after the purge step and before the injection step. The complementary filling step allows filling the lower space 4a with an accurate prescribed medical liquid volume 34, which was not possible in the first filling step due to the gas volume 32 that led to incorrect volume measures (usually based on the piston 36 course).

The process may include an injection step wherein the piston travels within the inner space along the longitudinal direction towards the lower end of the body, and wherein the medical liquid exits said lower space of the inner space to be injected into a medical tubing. As the evacuation path is closed, pressure increases within the lower space 4a when the piston 6 is pushed downward. When the pressure in the lower space 4a reaches the opening pressure of the second tubing valve 140, the second tubing valve 140 opens and the medical liquid 34 can exit the lower space 4a and travels the common line 102 to reach the patient line 116. The medical liquid can thus be injected without any gas. During the injection, the float 24 contacts the medical liquid.

It should be noted that the opening pressure threshold of the purge valve 22 (i.e. the first pressure threshold) is inferior to the opening pressure threshold (the second pressure threshold) of the second tubing valve 140 so that the purge valve 22 opens before the second tubing valve 140 opens when the gas is to be evacuated. During this injection step however, the obturation of the passage 38 by the selector (the float 24) means that the pressure increases in the lower space 4a but does not increase in the isolated intermediate portion 17b of the evacuation path. The purge valve 22 is thus kept in the blocking configuration.

As a variant as illustrated on **Figures 7a, 7b and 7c**, the selector can be a float 50 with a two-stage obturation of the evacuation path. The float 50 is configured to obturate the passage 38 with two distinct zones contacting the seat 40 defining the passage 38. To this end, the float 50 includes a first obturating part 50a and a second obturating part 50b, which are arranged so that when the float 50 goes upwards to obturate the passage 38, the first obturating part 50a first cooperates with the seat 40 for forming a first sealing, and then the second obturating part 50b cooperates with the seat 40 for forming a second sealing.

Preferably, the first obturating part 50a faces the passage 38, whereas the second obturating part 50b has a sealing portion 52 configured to cooperate with the seat 40 which surrounds the first obturating part 50a (in a projection on a plane perpendicular to the longitudinal direction). As a result, when the float 50 goes upwards, the second sealing surrounds the first sealing. In the depicted example, the first obturating part 50a of the float 50 can be as the obturating part described before and may be a ball as illustrated. The second obturating part 50b of the float 50 can correspond to the previously described floating part 24b of the float. The second obturating part 50b can still be enlarged with respect to the first obturating part 50a but now also have at least one sealing portion 52 protruding upwards around the upper portion. Preferably, the highest point of the sealing portion 52 is below the highest point of the first obturating part 50a, so that the first sealing occurs before the second sealing. It should be noted that, as before, the float 50 can be a one-piece float, even though a two-piece float is illustrated.

The seat 40 can be adapted to this kind of float 50. The seat 40 can define two wall portions of the passage 38, corresponding to a first seat 40a and to a second seat 40b. The second seat 40b surrounds the first seat 40a (in a projection on a plane perpendicular to the longitudinal direction). Preferably, the first seat 40a is deformable (for example with a hardness of 20-30 ShA (Shore A scale) to allow the float 50 to keep going upwards so that the float 50 can contact the second seat 40b after deformation of the first seat 40a.

In **Figure 7a****,** the float 50 is away from the seats 40a, 40b and does not obturate the passage 38. The gas can go through the passage 38 to be evacuated into the upper space 4b. In **Figure 7b****,** the float 50 begins to float on the medical liquid, and therefore travels upwards towards the seats 40a, 40b. The first obturating part 50a of the float contacts the first seat 40a, and causes the first sealing, as described above. The passage 38 is obturated by a first obturation. As the piston 6 still travels downwards, pressure increases in the cavity 36 of the lower portion 17a of the evacuation path, and medical liquid fills the cavity 36. The float 50 keeps pushing against the first seat 40a, which is deformed by the force exerted by the float 50. The float 50 can thus go upwards, and the sealing portion 52 of the second obturating part 50b contacts the second seat 40b. A second obturation occurs, as illustrated in **Figure 7c****.**

In this variant, a greater volume of gas is trapped below the obturated passage, and the second sealing ensures that no medical liquid will touch the first obturating part 50a, which is kept dry and devoid of any contaminant. The first obturating part 50a is kept clean and ensures that the first sealing occurs.

### The selector is a membrane

With reference to **Figures 8-11****,** a process for operating an injection device 110 with a membrane 60 as a selector is now described. The membrane 60 extends across the evacuation path, delimiting the lower portion 17a and the intermediate portion 17b of the evacuation path. The membrane 60 is hydrophobic and is configured to allow the gas to pass through while preventing the medical liquid from passing through. In this example, the membrane 60 is arranged in a cavity with a larger section (perpendicularly to the longitudinal direction) larger than the entrance 20 of the evacuation path. This is not necessary and the entrance 20 can have a larger section than the cavity 36. A larger cavity however increases the surface of the membrane 60 that can let the gas go through and therefore accelerates the purge.

In **Figure 8****,** the injection device 110 is shown before the purge, for instance during the filling step wherein the injection device 110 is filled with medical liquid and unwanted gas. During this filling step, the piston rod 12 is driven upwards, for example by the injector 108 acting on said piston rod 12, thereby causing the piston 6 to travel within the inner space 4 along the longitudinal direction towards the upper end of the body 2, i.e. upwards. The lower space 4a expands, resulting in a gas pressure within said lower space 4a to drop below the gas pressure within the upper space 4b, e.g. at the atmospheric pressure.

Since the membrane 60 allows gas to travel from the lower portion 17a to the intermediate portion 17b of the evacuation path, the decreased pressure in the lower space 4a is also found in the lower portion 17a and the intermediate portion 17b of the evacuation path. However, as the purge valve 22 requires an overpressure in the intermediate portion 17b of the evacuation path to be in the passing configuration, the purge valve 22 is kept in the blocking configuration. More precisely, the purge valve 22 is pressed downwards, sealing the vent 26 and thereby closing the evacuation path.

The expansion of the lower space 4a combined with the closing of the evacuation path by the purge valve 22 effectively creates a vacuum pressure in the lower space 4a, i.e. a pressure below the reference pressure. The pressure decreases until reaching the opening pressure of the first tubing valve 130, which is for example comprised between 0.2 and 0.5 bars below the reference pressure. The opening of the first tubing valve 130 creates an aspiration of the medical liquid to compensate this vacuum pressure in the lower space 4a. The medical liquid travels through the filling line 114 connected to the medical liquid container 104 to fill the lower space 4a. Gas present in the tubing is also aspirated into the lower space 4a.

Progressively, as the lower space 4a becomes filled with a volume 34 of medical liquid and a volume 32 of gas, the pressure within the lower space 4a increases and becomes closer to the atmospheric pressure. When the pressure within the lower space 4a reaches the closing pressure of the first tubing valve 130 (substantially similar to the opening pressure), the first tubing valve 130 closes and the filling is stopped. At the end of the filling step, the lower space 4a is filled with a volume 32 of gas above a volume of medical liquid 34. The gas pressure within the lower space 4a is still below the gas pressure within the upper space 4b because the pressure increase was stopped by the closing of the first tubing valve 130 before the vacuum pressure had been completely compensated. Consequently, the purge valve 22 stays in the blocking configuration.

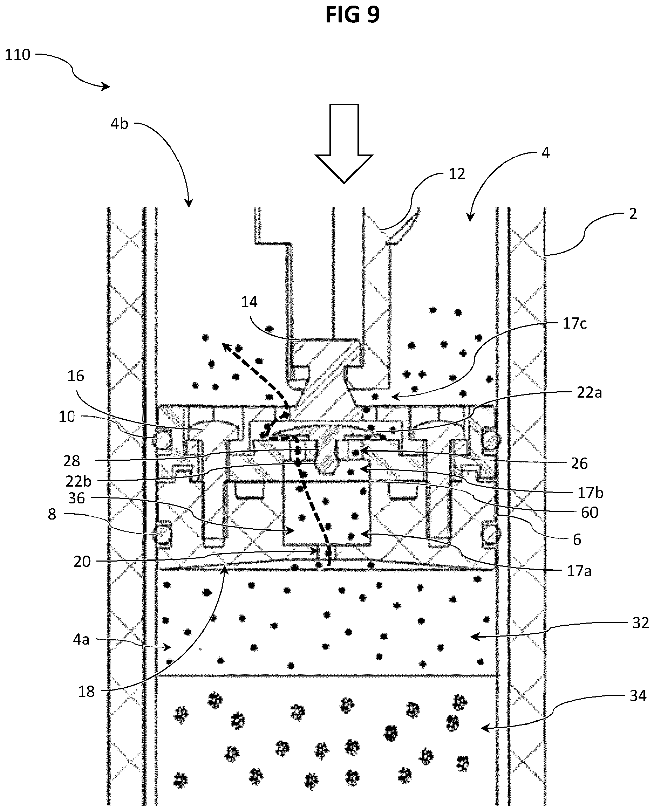

As explained above, after the filling of the injection device 110, the gas in the lower space 4a must be evacuated during a purge. This purge is performed by driving the piston 6 downwards, as shown on **Figure 9****.** The piston 6 travels within the inner space 4 along the longitudinal direction towards the lower end of the body 2. The lower space 4a shrinks, and gas pressure increases in the lower space 4a until the pressure difference between the gas pressure within the intermediate portion 17b, still communicating with the lower space 4a, and the gas pressure within the upper space 4b becomes higher than the opening pressure threshold of the purge valve 22, i.e. until the pressure difference reaches the first pressure threshold.

As indicated above, the purge valve 22 is configured to move to the passing configuration in response to an overpressure in the lower space 4a exceeding the reference pressure in the upper space 4b by at least the opening pressure threshold of the purge valve 22 (for example between 20 and 100 mbars of pressure difference between the overpressure and the reference pressure). The purge valve 22 therefore moves to the passing configuration, thereby opening the evacuation path. In the depicted example, the sealing disk 22a moves away from the vents 26, thereby unsealing said vents 26.

The gas is evacuated from the lower space 4a to the upper space 4b of the inner space 4 through the evacuation path traversing the piston 6. More specifically, the gas enters the evacuation path through the entrance 20, then travels along the lower portion 17a of the evacuation path, passes through the membrane 60, then travels along the intermediate portion 17b of the evacuation path, then through the vents 26 and finally along the upper portion 17c to reach the upper space 4b. This is shown by the dotted arrow in **Figure 9****.**

As the piston 6 travels downwards while the gas is evacuated through the evacuation path, the piston reaches the volume of medical liquid in the lower space 4a. More specifically, the lower interface 18 contacts the medical liquid, and the gas is pushed back towards the entrance 20 of the evacuation path since the entrance 20 opens in a highest portion of said lower interface 18. The gas is therefore evacuated from the lower space 4a before the medical liquid reaches the entrance 20 of the evacuation path. When all the gas has been evacuated, the medical liquid penetrates through the entrance 20 in the lower interface 18 of the piston 6 and fills the lower portion 17a of the evacuation path.

As illustrated, the membrane 60 can be arranged in a cavity 36, and the medical liquid begins filling the cavity 36, while the gas escapes the lower part of the cavity 36 which belongs to the lower portion 17a of the evacuation path to reach the upper part of the cavity 36, which belongs to the intermediate part 17b of the evacuation path. The membrane 60 is configured to allow the gas to pass through, while preventing the medical liquid from passing through. The membrane 60 is a hydrophobic tissue and may be made from, for example, PET (polyethylene terephthalate), ABS (acrylonitrile butadiene styrene), PA (polyamide) or other plastic material, or medical stainless steel. The membrane 60 may be coated, for example, with PTFE (polytetrafluoroethylene), ePTFE (expanded polytetrafluoroethylene) or other hydrophobic raw material.

The membrane 60 may be characterized by its air permeability and the size of its pore. In particular, the membrane 60 allows for very low air permeability, such as 5 l/m²/sec at 120 Pa.s or lower. The low air permeability allows for small pre sizes in the membrane. For example, the size of the pores of the membrane 60 may be in the range of approximately between 0.01 and 0.25 microns. Optionally, the membrane 60 may have small pores measuring 0.07 microns or smaller. For example, the thickness of the tissue of the membrane 60 may be about 200 microns.

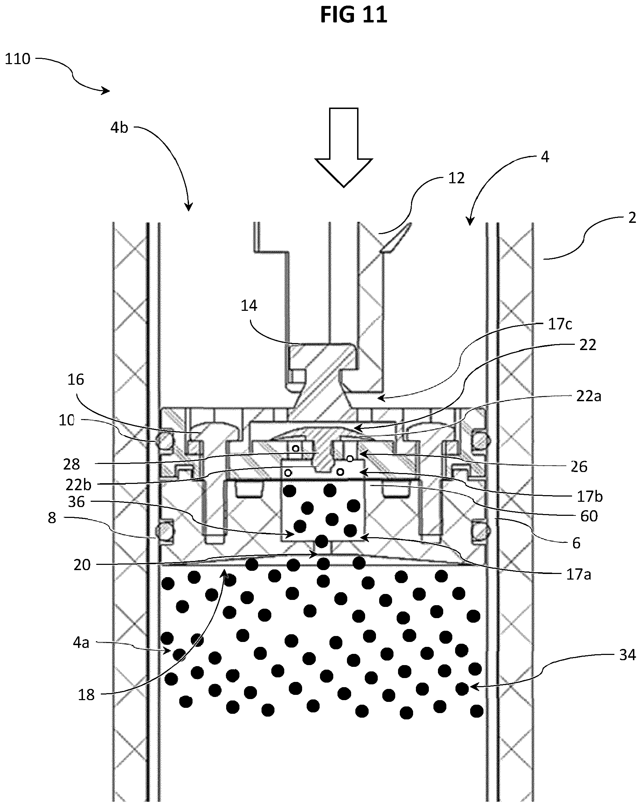

When the medical liquid contacts the membrane 60, the membrane 60 prevents the medical liquid from passing through, and the medical liquid is blocked in the lower part of the cavity 36 which belongs to the lower portion 17a of the evacuation path. As the gas can still escape the intermediate portion 17b of the evacuation path to reach the upper space 4b while gas no longer arrives in said intermediate portion 17b from the lower portion 17a, the pressure in the intermediate portion 17b of the evacuation path decreases until the difference between the pressure in the intermediate portion 17b and the reference pressure reaches the closing pressure threshold of the purge valve 22, which is slightly above the reference pressure since the upper space 4b is at said reference pressure. For example, the closing pressure threshold of the purge valve 22 can correspond to a positive pressure difference of 20 to 100 mbars between the pressure within the intermediate portion 17b and the reference pressure. Preferably, the closing pressure threshold and the opening closing threshold are substantially the same, but they can also differ. As a result, the purge valve 22 is now closed **(****Figure 11****),** the sealing disk 22a covering the vent 26. The purge is now over.

The process may comprise a complementary filling step which occurs after the purge step and before the injection step. The complementary filling step allows filling the lower space 4a with an accurate prescribed medical liquid volume 34, which was not possible in the first filling step due to the gas volume 32 that led to incorrect volume measures (usually based on the piston 36 course).

The process may include an injection step wherein the piston travels within the inner space along the longitudinal direction towards the lower end of the body, and wherein the medical liquid exits said lower space of the inner space to be injected into a medical tubing. As the evacuation path is closed, pressure increases within the lower space 4a when the piston 6 is pushed downward. When the pressure in the lower space 4a reaches the opening pressure of the second tubing valve 140 the second tubing valve 140 opens and the medical liquid 34 can exit the lower space 4a and travels the common line 102 to reach the patient line 116. The medical liquid can thus be injected without any gas. It should be noted that the opening pressure threshold of the purge valve 22 (i.e. the first pressure threshold) is inferior to the opening pressure threshold (the second pressure threshold) of the second tubing valve 140 so that the purge valve 22 opens before the second tubing valve 140 opens when the gas is to be evacuated. The purge valve 22 is thus kept in the blocking configuration during the injection step.

During the injection, the membrane 60 contacts the medical liquid and is subjected to the whole injection pressure due to the contact with the medical liquid. The injection pressure can reach values such as 24 bar for an injection rate of 10 cc/second. The membrane 60 is selected to sustain such high pressure. To help the membrane 60 withstanding the mechanical force exerted by the medical fluid, a support organ can be arranged in the upper part of the cavity 36, i.e. in the intermediate portion 17b of the evacuation path.

The support organ is held against an upper side of the membrane 60 to mechanically support the membrane 60. The support organ is permeable to the gas (e.g. air) and of course allows the gas passes through the membrane 60 and through said support organ. The support may be made from a semi-rigid or rigid foam. Optionally, the support may be a plastic porous plate or micro-hold metallic piece. For example, the support organ can be a foam, a mesh or a lattice, preferably made of metal. It can however be of any type as long as it provides a mechanical support for the membrane without preventing the gas from passing through the intermediate portion 17b of the evacuation path.

While the present invention has been described with respect to certain preferred embodiments, it is obvious that it is in no way limited thereto and it comprises all the technical equivalents of the means described and their combinations.

## Claims

1. An injection device (1) for injecting into a medical tubing a medical liquid from a medical liquid container, said injection device comprising:
- a body (2) defining an inner space (4) extending in a longitudinal direction between an upper end of the body (2) and a lower end of the body (2), the lower end of the body comprising a medical tubing interface (112) through which the medical liquid can penetrate the inner space (4) from the medical liquid container and can exit said inner space (4) to be injected into the medical tubing (112),
- a piston (6) arranged within the inner space (4) and configured for travelling within the inner space (4) along the longitudinal direction, said piston (6) delimiting an upper space (4b) and a lower space (4a) of the inner space, said lower space (4a) configured to receive the medical liquid,
- an evacuation path, said evacuation path traversing the piston (6) in the longitudinal direction from the lower space (4a) to the upper space (4b) of the inner space,
**characterized in that** the injection device (1) further comprises:
- a selector (24, 50, 60) arranged within the evacuation path between a lower portion (17a) of the evacuation path and an intermediate portion (17b) of the evacuation path, said lower portion (17a) of the evacuation path being connected to the lower space (4a), wherein the selector (24, 50, 60) is configured for selectively allowing gas to go through said selector and to travel along the evacuation path from the lower portion (17a) to the intermediate portion (17b) of the evacuation path, said selector (24, 50, 60) configured for selectively preventing the medical liquid from going through said selector (24, 50, 60) and from travelling along the evacuation path from the lower space (4a) to the upper space (4b) of the inner space,
- a purge valve (22) arranged in the evacuation path between the intermediate portion (17b) of the evacuation path and an upper portion (17c) of the evacuation path and configured for moving between a blocking configuration in which the purge valve (22) closes the evacuation path and a passing configuration in which the purge valve (22) keeps open said evacuation path, wherein the passing configuration of the purge valve (22) requires an overpressure in the intermediate portion (17b) of the evacuation path caused by the piston (6) travelling towards the lower end of the body (2).

2. The injection device (110) according to claim 1, wherein the purge valve (22) is configured for being in the blocking configuration when the piston (6) travels towards the upper end of the body (2).

3. The injection device (110) of any one of the preceding claims, wherein the lower portion (17a) of the evacuation path is configured for receiving both medical liquid and a gas, and the intermediate portion (17b) and the upper portion (17c) of the evacuation path are configured for receiving only gas.

4. The injection device (110) of any one of the preceding claims, wherein the upper space (4b) is configured to be kept at a constant reference pressure and the overpressure in the intermediate portion of the evacuation path corresponds to a gas pressure above the reference pressure.

5. The injection device (110) of any one of the preceding claims, wherein the piston comprises a lower interface (18) delimiting the lower space (4a) of the inner space (4), said lower interface (18) comprising an entrance (20) of the evacuation path which opens in a highest portion of said lower interface.

6. The injection device (110) of the preceding claim, wherein the lower interface (18) has a convex surface seen from the lower space (4a) of the inner space and the entrance (20) of the evacuation path opens in a middle of said lower interface (18) or the lower interface (18) has a concave surface seen from the lower space (4a) of the inner space and the entrance (20) of the evacuation path opens in a periphery of said lower interface (18).

7. The injection device (110) of any one of the preceding claims, wherein the selector (24, 50, 60) is a float (24, 50) configured for floating on the medical liquid and arranged in a cavity (36), said cavity (36) comprising at least a passage (38) which belongs to the evacuation path, wherein said float (24, 50) is configured for travelling within said cavity (36) along the longitudinal direction between a blocking configuration in which the float (24, 50) obturates the passage (38), thereby closing the evacuation path, and an open configuration in which the float (24, 50) is spaced apart from the passage (38), thereby letting open the evacuation path.

8. The injection device (110) of the preceding claim, wherein the float (24) has at least one obturating part (24a) and a floating part (24b), said obturating part configured to obturate the passage (38), wherein the floating part (24b) has an enlarged section with respect to a widest section of the obturating part (24a).

9. The injection device (110) of any one of claims 7 to 8, wherein the float (50) is configured to obturate the passage (38) with two distinct zones (50a, 50b) contacting a seat (40) defining the passage (38).

10. The injection device (110) of any one of claims 1 to 6, wherein the selector is a membrane (60) extending across the evacuation path, said membrane (60) being hydrophobic and configured for letting the gas passes through, and for preventing the medical liquid from passing through.

11. The injection device (110) of claim 10, wherein the membrane (60) is arranged in a cavity (36), and a support organ is arranged in said cavity (36) against an upper side of the membrane to mechanically support the membrane, said support organ allowing the gas passes through the membrane and through said support organ.

12. An injection system (100) comprising:
- an injection device (110) as claimed in any one of the preceding claims,
- a first connector (104) configured to be connected to a medical liquid container (104),
- a medical liquid supply line (114) configured to connect the first connector (104) to the medical tubing interface (112) for supplying the medical liquid to the injection system,
- a common line (102) configured to be connected to the medical tubing interface (112) and to a patient line (116) for injecting the medical liquid into the patient line.

13. The injection system (100) of the previous claim, wherein the overpressure corresponds to a pressure in the intermediate portion (17b) of the evacuation path exceeding a reference pressure in the upper space (4b) by at least a first pressure threshold, and
wherein the common line (102) comprises a threshold valve (140) with a second pressure threshold,
wherein the first pressure threshold is inferior to the second pressure threshold.

14. A process for operating an injection device as claimed in any one of claims 1-11, wherein the injection device (110) is kept with the upper end of the body (2) upwards, and the lower end of the body (2) downwards, the process comprising:
- a filling step, wherein the piston (6) travels within the inner space (4) along the longitudinal direction towards the upper end of the body (2), causing the medical liquid from the medical liquid container (104) and gas to penetrate the inner space (4) through the medical tubing interface (112), wherein the purge valve (22) is in the blocking configuration,
- a purge step, wherein the piston (6) travels within the inner space (4) along the longitudinal direction towards the lower end of the body (2), and wherein the gas is evacuated from the lower space (4a) to the upper space (4b) of the inner space (4) through the evacuation path traversing the piston (6), while the selector (24, 50, 60) keeps the medical liquid in the lower space (4a), wherein the purge valve (22) is in the passing configuration.

## Patentansprüche

1. Injektionsvorrichtung (1) zum Injizieren einer medizinischen Flüssigkeit von einem medizinischen Flüssigkeitsbehälter in einen medizinischen Schlauch, wobei die Injektionsvorrichtung umfasst:
- einen Körper (2), der einen Innenraum (4) definiert, der sich in einer Längsrichtung zwischen einem oberen Ende des Körpers (2) und einem unteren Ende des Körpers (2) erstreckt, wobei das untere Ende des Körpers eine medizinische Schlauchschnittstelle (112) umfasst, durch welche die medizinische Flüssigkeit von dem medizinischen Flüssigkeitsbehälter in den Innenraum (4) eindringen kann und von dem Innenraum (4) austreten kann, um in den medizinischen Schlauch (112) injiziert zu werden,
- einen Kolben (6), der innerhalb des Innenraums (4) eingerichtet ist und dazu ausgestaltet ist, sich innerhalb des Innenraums (4) entlang der Längsrichtung zu bewegen, wobei der Kolben (6) einen oberen Raum (4b) und einen unteren Raum (4a) des Innenraums abgrenzt, wobei der untere Raum (4a) dazu ausgestaltet ist, die medizinische Flüssigkeit aufzunehmen,
- einen Abführungsweg, wobei der Abführungsweg den Kolben (6) in der Längsrichtung vom unteren Raum (4a) zum oberen Raum (4b) des Innenraums durchquert,
**dadurch gekennzeichnet, dass** die Injektionsvorrichtung (1) ferner umfasst:
- eine Auswahleinrichtung (24, 50, 60), die innerhalb des Abführungswegs zwischen einem unteren Abschnitt (17a) des Abführungswegs und einem Zwischenabschnitt (17b) des Abführungswegs eingerichtet ist, wobei der untere Abschnitt (17a) des Abführungswegs mit dem unteren Raum (4a) verbunden ist, wobei die Auswahleinrichtung (24, 50, 60) dazu ausgestaltet ist, es selektiv Gas zu erlauben, die Auswahleinrichtung zu durchqueren und sich entlang des Abführungswegs vom unteren Abschnitt (17a) zum Zwischenabschnitt (17b) des Abführungswegs zu bewegen, wobei die Auswahleinrichtung (24, 50, 60) dazu ausgestaltet ist, selektiv zu verhindern, dass die medizinische Flüssigkeit die Auswahleinrichtung (24, 50, 60) durchquert und sich entlang des Abführungswegs vom unteren Raum (4a) zum oberen Raum (4b) des Innenraums bewegt,
- ein Ablassventil (22), das in dem Abführungsweg zwischen dem Zwischenabschnitt (17b) des Abführungswegs und einem oberen Abschnitt (17c) des Abführungswegs eingerichtet ist und dazu ausgestaltet ist, sich zwischen einer Sperrausgestaltung, in der das Ablassventil (22) den Abführungsweg schließt, und einer Durchgangsausgestaltung zu bewegen, in der das Ablassventil (22) den Abführungsweg offen lässt, wobei die Durchgangsausgestaltung des Ablassventils (22) einen Überdruck in dem Zwischenabschnitt (17b) des Abführungswegs erfordert, der durch die Bewegung des Kolbens (6) hin zum unteren Ende des Körpers (2) bewirkt wird.

2. Injektionsvorrichtung (110) nach Anspruch 1, wobei das Ablassventil (22) dazu ausgestaltet ist, in der Sperrausgestaltung zu sein, wenn der Kolben (6) sich hin zum oberen Ende des Körpers (2) bewegt.

3. Injektionsvorrichtung (110) nach einem der vorhergehenden Ansprüche, wobei der untere Abschnitt (17a) des Abführungswegs dazu ausgestaltet ist, sowohl medizinische Flüssigkeit als auch ein Gas aufzunehmen, und der Zwischenabschnitt (17b) und der obere Abschnitt (17c) des Abführungswegs dazu ausgestaltet sind, lediglich Gas aufzunehmen.

4. Injektionsvorrichtung (110) nach einem der vorhergehenden Ansprüche, wobei der obere Raum (4b) dazu ausgestaltet ist, bei einem konstanten Bezugsdruck gehalten zu werden, und der Überdruck in dem Zwischenabschnitt des Abführungswegs einem Gasdruck über dem Bezugsdruck entspricht.

5. Injektionsvorrichtung (110) nach einem der vorhergehenden Ansprüche, wobei der Kolben eine untere Grenzfläche (18) umfasst, die den unteren Raum (4a) des Innenraums (4) abgrenzt, wobei die untere Grenzfläche (18) einen Eingang (20) des Abführungswegs umfasst, der sich in einem höchsten Abschnitt der unteren Grenzfläche öffnet.

6. Injektionsvorrichtung (110) nach dem vorhergehenden Anspruch, wobei die untere Grenzfläche (18), von dem unteren Raum (4a) des Innenraums betrachtet, eine konvexe Oberfläche aufweist und der Eingang (20) des Abführungswegs sich in einer Mitte der unteren Grenzfläche (18) öffnet oder die untere Grenzfläche (18), vom unteren Raum (4a) des Innenraums betrachtet, eine konkave Oberfläche aufweist und der Eingang (20) des Abführungswegs sich in einer Peripherie der unteren Grenzfläche (18) öffnet.

7. Injektionsvorrichtung (110) nach einem der vorhergehenden Ansprüche, wobei die Auswahleinrichtung (24, 50, 60) ein Schwimmer (24, 50) ist, der dazu ausgestaltet ist, auf der medizinischen Flüssigkeit zu schwimmen, und in einem Hohlraum (36) eingerichtet ist, wobei der Hohlraum (36) mindestens einen Durchgang (38) umfasst, der zu dem Abführungsweg gehört, wobei der Schwimmer (24, 50) dazu ausgestaltet ist, sich innerhalb des Hohlraums (36) entlang der Längsrichtung zwischen einer Sperrausgestaltung, in welcher der Schwimmer (24, 50) den Durchgang (38) verschließt, wodurch der Abführungsweg geschlossen wird, und einer offenen Ausgestaltung zu bewegen, in welcher der Schwimmer (24, 50) von dem Durchgang (38) beabstandet ist, wodurch der Abführungsweg offen gelassen wird.

8. Injektionsvorrichtung (110) nach dem vorhergehenden Anspruch, wobei der Schwimmer (24) mindestens einen verschließenden Teil (24a) und einen schwimmenden Teil (24b) aufweist, wobei der verschließende Teil dazu ausgestaltet ist, den Durchgang (38) zu verschließen, wobei der schwimmende Teil (24b) einen in Bezug auf einen breitesten Querschnitt des verschließenden Teils (24a) vergrößerten Querschnitt aufweist.

9. Injektionsvorrichtung (110) nach einem der Ansprüche 7 bis 8, wobei der Schwimmer (50) dazu ausgestaltet ist, den Durchgang (38) mit zwei getrennten Zonen (50a, 50b) zu verschließen, die einen Sitz (40) berühren, der den Durchgang (38) definiert.

10. Injektionsvorrichtung (110) nach einem der Ansprüche 1 bis 6, wobei die Auswahleinrichtung eine Membran (60) ist, die sich durch den Abführungsweg erstreckt, wobei die Membran (60) wasserabweisend ist und dazu ausgestaltet ist, den Durchgang des Gases zuzulassen und den Durchgang der medizinischen Flüssigkeit zu verhindern.

11. Injektionsvorrichtung (110) nach Anspruch 10, wobei die Membran (60) in einem Hohlraum (36) eingerichtet ist und ein Unterstützungsorgan in dem Hohlraum (36) gegen eine obere Seite der Membran angeordnet ist, um die Membran mechanisch zu unterstützen, wobei das Unterstützungsorgan es dem Gas erlaubt, die Membran und das Unterstützungsorgan zu durchqueren.

12. Injektionssystem (100), umfassend:
- eine Injektionsvorrichtung (110) nach einem der vorhergehenden Ansprüche,
- einen ersten Verbinder (104), der dazu ausgestaltet ist, mit einem medizinischen Flüssigkeitsbehälter (104) verbunden zu sein,
- eine medizinische Flüssigkeitszufuhrleitung (114), die dazu ausgestaltet ist, den ersten Verbinder (104) mit der medizinischen Schlauchschnittstelle (112) zum Zuführen der medizinischen Flüssigkeit zum Injektionssystem zu verbinden,
- eine gemeinsame Leitung (102), die dazu ausgestaltet ist, mit der medizinischen Schlauchschnittstelle (112) und mit einer Patientenleitung (116) zum Injizieren der medizinischen Flüssigkeit in die Patientenleitung verbunden zu sein.

13. Injektionssystem (100) nach dem vorhergehenden Anspruch, wobei der Überdruck dem Überschreiten eines Bezugsdrucks in dem oberen Raum (4b) durch einen Druck in dem Zwischenabschnitt (17b) des Abführungswegs um mindestens einen ersten Druckschwellenwert entspricht, und
wobei die gemeinsame Leitung (102) ein Schwellenwertventil (140) mit einem zweiten Druckschwellenwert umfasst,
wobei der erste Druckschwellenwert niedriger als der zweite Druckschwellenwert ist.

14. Verfahren für die Betätigung einer Injektionsvorrichtung nach einem der Ansprüche 1 bis 11, wobei die Injektionsvorrichtung (110) mit dem oberen Ende des Körpers (2) nach oben und dem unteren Ende des Körpers (2) nach unten gehalten wird, wobei der Prozess umfasst:
- einen Füllschritt, wobei der Kolben (6) sich innerhalb des Innenraums (4) entlang der Längsrichtung hin zum oberen Ende des Körpers (2) bewegt, wodurch bewirkt wird, dass die medizinische Flüssigkeit von dem medizinischen Flüssigkeitsbehälter (104) und Gas durch die medizinische Schlauchschnittstelle (112) in den Innenraum (4) eindringen, wobei das Ablassventil (22) sich in der Sperrausgestaltung befindet,
- einen Ablassschritt, wobei der Kolben (6) sich innerhalb des Innenraums (4) entlang der Längsrichtung hin zum unteren Ende des Körpers (2) bewegt, und wobei das Gas von dem unteren Raum (4a) zum oberen Raum (4b) des Innenraums (4) durch den Abführungsweg, der den Kolben durchquert (6), abgeführt wird, während die Auswahleinrichtung (24, 50, 60) die medizinische Flüssigkeit im unteren Raum (4a) hält, wobei das Ablassventil (22) sich in der Durchgangsausgestaltung befindet.

## Revendications

1. Dispositif d'injection (1) pour injecter dans une tubulure médicale un liquide médical provenant d'un réservoir de liquide médical, ledit dispositif d'injection comprenant :
- un corps (2) définissant un espace intérieur (4) s'étendant suivant une direction longitudinale entre une extrémité supérieure du corps (2) et une extrémité inférieure du corps (2), l'extrémité inférieure du corps comprenant une interface de tubulure médicale (112) à travers laquelle le liquide médical peut pénétrer dans l'espace intérieur (4) à partir du récipient de liquide médical et peut sortir dudit espace intérieur (4) pour être injecté dans la tubulure médicale (112),
- un piston (6) disposé dans l'espace intérieur (4) et configuré pour se déplacer dans l'espace intérieur (4) suivant la direction longitudinale, ledit piston (6) délimitant un espace supérieur (4b) et un espace inférieur (4a) de l'espace intérieur, ledit espace inférieur (4a) étant configuré pour recevoir le liquide médical,
- une voie d'évacuation, ladite voie d'évacuation traversant le piston (6) suivant la direction longitudinale depuis l'espace inférieur (4a) vers l'espace supérieur (4b) de l'espace intérieur,
**caractérisé en ce que** le dispositif d'injection (1) comprend en outre :
- un sélecteur (24, 50, 60) disposé à l'intérieur de la voie d'évacuation entre une partie inférieure (17a) de la voie d'évacuation et une partie intermédiaire (17b) de la voie d'évacuation, ladite partie inférieure (17a) de la voie d'évacuation étant reliée à l'espace inférieur (4a), dans lequel le sélecteur (24, 50, 60) est configuré pour permettre sélectivement au gaz de passer à travers ledit sélecteur et de se déplacer le long de la voie d'évacuation depuis la partie inférieure (17a) vers la partie intermédiaire (17b) de la voie d'évacuation, ledit sélecteur (24, 50, 60) étant configuré pour empêcher sélectivement le liquide médical de passer à travers ledit sélecteur (24, 50, 60) et de se déplacer le long de la voie d'évacuation depuis l'espace inférieur (4a) vers l'espace supérieur (4b) de l'espace intérieur,
- une vanne de purge (22) agencée sur la voie d'évacuation entre la partie intermédiaire (17b) de la voie d'évacuation et une partie supérieure (17c) de la voie d'évacuation et configurée pour se déplacer entre une configuration de blocage dans laquelle la vanne de purge (22) ferme la voie d'évacuation et une configuration de passage dans laquelle la vanne de purge (22) maintient ladite voie d'évacuation ouverte, dans laquelle la configuration de passage de la vanne de purge (22) nécessite une surpression dans la partie intermédiaire (17b) de la voie d'évacuation provoquée par un déplacement du piston (6) vers l'extrémité inférieure du corps (2).

2. Dispositif d'injection (110) selon la revendication 1, dans lequel la vanne de purge (22) est configurée pour être en configuration de blocage lorsque le piston (6) se déplace vers l'extrémité supérieure du corps (2).

3. Dispositif d'injection (110) selon l'une quelconque des revendications précédentes, dans lequel la partie inférieure (17a) de la voie d'évacuation est configurée pour recevoir à la fois le liquide médical et un gaz, et la partie intermédiaire (17b) et la partie supérieure (17c) de la voie d'évacuation sont configurées pour recevoir uniquement du gaz.

4. Dispositif d'injection (110) selon l'une quelconque des revendications précédentes, dans lequel l'espace supérieur (4b) est configuré pour être maintenu à une pression de référence constante et la surpression dans la partie intermédiaire de la voie d'évacuation correspond à une pression de gaz supérieure à la pression de référence.

5. Dispositif d'injection (110) selon l'une quelconque des revendications précédentes, dans lequel le piston comprend une interface inférieure (18) délimitant l'espace inférieur (4a) de l'espace intérieur (4), ladite interface inférieure (18) comprenant une entrée (20) de la voie d'évacuation qui s'ouvre dans une partie sommitale de ladite interface inférieure.

6. Dispositif d'injection (110) selon la revendication précédente, dans lequel l'interface inférieure (18) présente une surface convexe vue de l'espace inférieur (4a) de l'espace intérieur et l'entrée (20) de la voie d'évacuation s'ouvre au milieu de ladite interface inférieure (18) ou l'interface inférieure (18) présente une surface concave vue de l'espace inférieur (4a) de l'espace intérieur et l'entrée (20) de la voie d'évacuation s'ouvre à la périphérie de ladite interface inférieure (18).

7. Dispositif d'injection (110) de l'une quelconque des revendications précédentes, dans lequel le sélecteur (24, 50, 60) est un flotteur (24, 50) configuré pour flotter sur le liquide médical et disposé dans une cavité (36), ladite cavité (36) comprenant au moins un passage (38) qui appartient à la voie d'évacuation, dans lequel ledit flotteur (24, 50) est configuré pour se déplacer à l'intérieur de ladite cavité (36) suivant la direction longitudinale entre une configuration de blocage dans laquelle le flotteur (24, 50) obture le passage (38), fermant ainsi la voie d'évacuation, et une configuration ouverte dans laquelle le flotteur (24, 50) est écarté du passage (38), laissant ainsi ouverte la voie d'évacuation.

8. Dispositif d'injection (110) selon la revendication précédente, dans lequel le flotteur (24) comporte au moins une partie obturatrice (24a) et une partie flottante (24b), ladite partie obturatrice étant configurée pour obturer le passage (38), la partie flottante (24b) ayant une section élargie par rapport à la section la plus large de la partie obturatrice (24a).

9. Dispositif d'injection (110) selon l'une quelconque des revendications 7 à 8, dans lequel le flotteur (50) est configuré pour obturer le passage (38) avec deux zones distinctes (50a, 50b) en contact avec un siège (40) définissant le passage (38).

10. Dispositif d'injection (110) selon l'une quelconque des revendications 1 à 6, dans lequel le sélecteur est une membrane (60) s'étendant en travers de la voie d'évacuation, ladite membrane (60) étant hydrophobe et configurée pour laisser passer le gaz et empêcher le liquide médical de passer au travers.

11. Dispositif d'injection (110) selon la revendication 10, dans lequel la membrane (60) est disposée dans une cavité (36), et un organe de support est disposé dans ladite cavité (36) contre un côté supérieur de la membrane pour soutenir mécaniquement la membrane, ledit organe de support permettant au gaz de passer à travers la membrane et à travers ledit organe de support.

12. Système d'injection (100) comprenant
- un dispositif d'injection (110) selon l'une quelconque des revendications précédentes,
- un premier connecteur (104) configuré pour être connecté à un réservoir de liquide médical (104),
- une ligne d'alimentation en liquide médical (114) configurée pour connecter le premier connecteur (104) à l'interface de tubulure médicale (112) pour l'alimentation du système d'injection en liquide médical,
- une ligne commune (102) configurée pour être connectée à l'interface de tubulure médicale (112) et à une ligne de patient (116) pour injecter le liquide médical dans la ligne patient.

13. Système d'injection (100) selon la revendication précédente, dans lequel la surpression correspond à une pression dans la partie intermédiaire (17b) de la voie d'évacuation dépassant une pression de référence dans l'espace supérieur (4b) d'au moins un premier seuil de pression, et
dans lequel la conduite commune (102) comprend une soupape de seuil (140) avec un second seuil de pression,
dans lequel le premier seuil de pression est inférieur au second seuil de pression.

14. Procédé de fonctionnement d'un dispositif d'injection selon l'une quelconque des revendications 1 à 11, dans lequel le dispositif d'injection (110) est maintenu avec l'extrémité supérieure du corps (2) vers le haut et l'extrémité inférieure du corps (2) vers le bas, le procédé comprenant :
- une étape de remplissage, dans laquelle le piston (6) se déplace dans l'espace intérieur (4) suivant de la direction longitudinale vers l'extrémité supérieure du corps (2), provoquant la pénétration dans l'espace intérieur (4), à travers la tubulure médicale (112), du liquide médical provenant du réservoir de liquide médical (104) et de gaz, la valve de purge (22) étant en configuration de blocage,
- une étape de purge, dans laquelle le piston (6) se déplace dans l'espace intérieur (4) suivant la direction longitudinale vers l'extrémité inférieure du corps (2), et dans laquelle le gaz (20) est évacué de l'espace inférieur (4a) vers l'espace supérieur (4b) de l'espace intérieur (4) par la voie d'évacuation traversant le piston (6), tandis que le sélecteur (24, 50, 60) maintient le liquide médical dans l'espace inférieur (4a), la vanne de purge (22) étant en configuration de passage.
